# EUROPEAN PATENT APPLICATION

(11) **EP 4 491 103 A1**
(43) Date of publication of application: **15.01.2025**
(21) Application number: 23386062.6
(22) Date of filing: 13.07.2023
(51) Int. Cl.: A61B 5/00, A61B 5/024, A61B 5/08

(54) **INTELLIGENT HOSPITAL BEDS FOR PATIENT CARE**

(30) Priority: 12.07.2023 GR 20230100571
(71) Applicant: VIDAVO S.A., 57001 Thessaloniki (GR)
(72) Inventor: ANGELIDIS, Pantelis, 57001 (GR); ATHANASIADIS, Panagiotis, 15771 Zografou, Attiki (GR)

(57) **Abstract**

The invention concerns an innovative system for monitoring and assessing a patient's condition during their hospital stay while bedridden. It aims to upgrade a standard hospital bed to an intelligent patient care station, by utilizing a network of sensors and a mattress cover embedded with micro-sensors. The invention measures the surface pressures arising from the patient's body position, allowing the system to infer parameters such as heart rate, respiratory rate, patient position, and body pressure at different sites. Moreover, it measures the humidity levels under the bedridden patient's body as well as their temperature. This real-time data is displayed via a user-friendly interface on a bed-adaptive touchscreen and processed through medical rules and intelligent algorithms, enabling early detection of potential pathological conditions and pressure ulcers. The invention sends data and early warning notifications to a cloud server and portable devices for immediate clinical intervention. The ultimate objective of this innovative system is to improve the quality of hospitalization by providing continuous monitoring and assessment of the patient's condition and eliminate the possibility of developing pressure ulcers.

## Description

The present invention is a comprehensive bedridden patient monitoring system designed to enhance patient care during hospital stays. The system's distinguishing feature is its capacity to collect data from multiple sensors and use specialized algorithms to extract pertinent information about a patient's health status. It can predict the likelihood of pressure ulcer development and alert healthcare professionals about potential health deterioration.

The system incorporates a specifically developed software that undertakes the task of collecting and visualizing all data from the sensors. This software development was carried out on a Raspberry Pi 4 (RPi4) device within a Linux environment, and the visualization and/or user interface is implemented on a 13-inch touch screen.

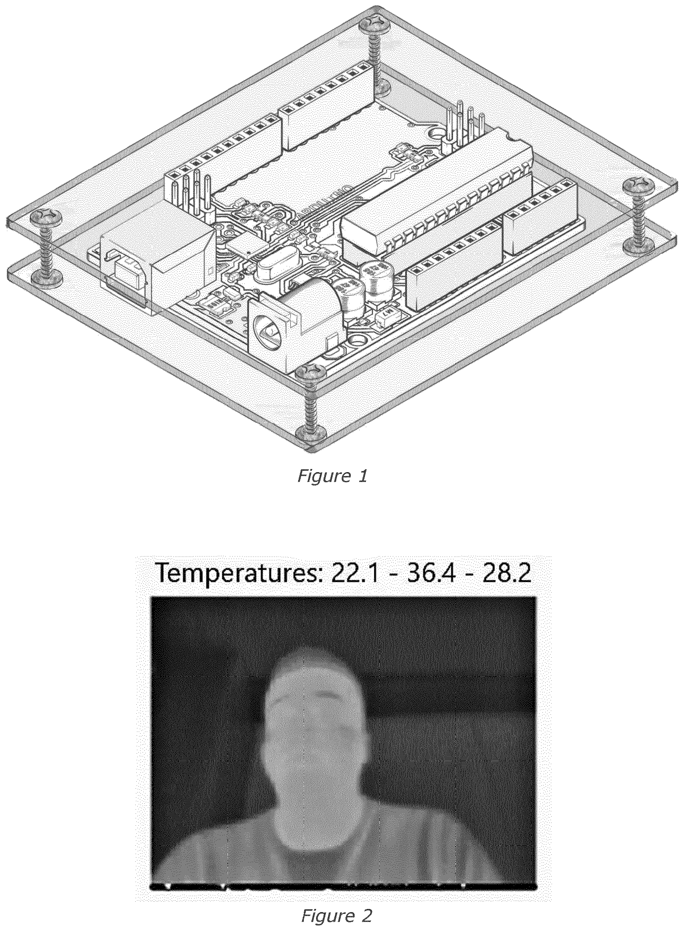

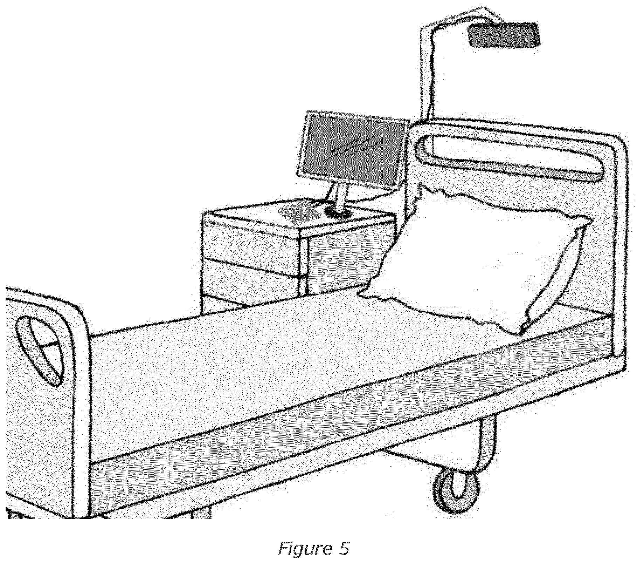

In addition, a prototype electronic circuit has been designed for use on an Arduino UNO (shield) as a management unit for analog signals from the respective sensors. This unit is connected to the central unit (RPi4), where it transmits the measurements obtained from the sensors. This two-tier architecture/set-up (Figure 1, Figure 5(4)) allows for reliable, real-time data collection and processing, central to the early identification of patient health risks and effective clinical intervention.

The system comprises several key components, including a pressure-sensitive mattress, the Mattress Mat Dev Kit 1.9 by Sensing Tex. This mattress generates a detailed pressure/force map that spans the entire area of the patient's bed. The pressure map is presented as a grid of 1056 values (48x22), updated at a rate of up to four times per second, enabling the system to determine the patient's body position and the distribution of pressure across different body sites.

The system also includes a thermal camera, the FLC1 Body Temperature Camera by DERMALOG (2020) (Figure 5(2)). The camera is designed to accurately measure body temperature, providing an additional vital sign for the system to monitor and assess. It is connected to the RPi4 via a USB port. In practice, it is designed to be placed at a point directly above the patient's head on a stable base. The camera's range extends up to two meters. It produces grayscale images with 16-bit values representing temperature values which are converted into a bitmap image with a grayscale color scheme where the value 0°C corresponds to black, and the value 50°C corresponds to white. Figure 2 shows a frame taken from the camera pointed towards a person. From left to right the values are the minimum, maximum, and average temperature, all given in degrees Celsius. As we observe, the maximum temperature corresponds to typical temperature values from the facial area, where temperatures in the range of 35-37°C are expected. The thermal camera provides an important means for constant, non-contact monitoring of the patient's body temperature, enhancing the system's ability to assess the patient's condition in real-time.

Another important component of the system is the humidity sensor ELES-01S (incontinence sensor) which is designed to detect abnormal humidity levels in the bed, covering an area of dimensions 75x45cm, sufficient for the pelvis region. Humidity levels are essential in maintaining patient comfort and are key in predicting the risk of pressure ulcer development. The sensor comprises a rectangular piece of cotton fabric, measuring 75x45cm, onto which two electrodes made of conductive thread/wire have been sewn, covering a significant portion of the fabric area. The sensor operates like an ohmic resistor, which can be directly measured with an ohmmeter at the ends of the electrodes. Between the electrodes, there is only fabric, which acts as an insulator, thus the resistance is practically infinite. However, when a liquid solution containing electrolytes, such as tap water or bodily fluids, is present, the resistance of the damp fabric decreases, or alternatively, its conductivity increases. The measurement of the resistance via the electrodes provides information about the amount of humidity in the sensor. To ensure the proper operation of the sensor, a calibration process was conducted. This calibration allows the sensor to provide accurate measurements of humidity levels, essential for detecting unusual moisture levels in the bed, which could signal the risk of developing pressure ulcers.

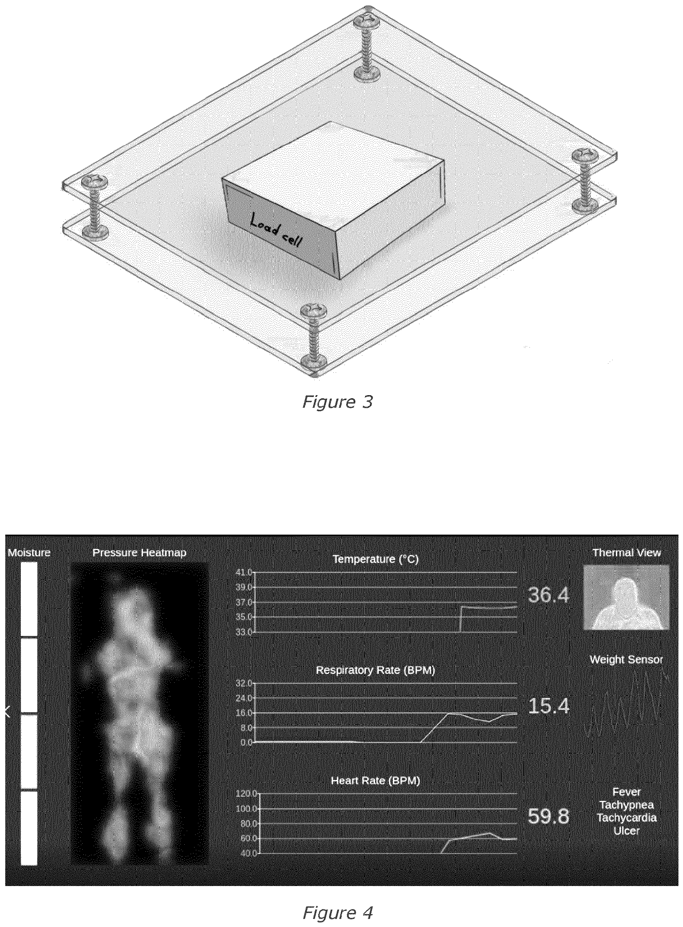

Calculation of the heart rate and respiratory rate was performed utilizing a load cell with a weight capacity of 50Kg by SparkFun Electronics (2022). The load cell employs strain gauges, and its construction can be discerned in Figure 3. For the placement, two acrylic plates were used, between which the load cell was fitted. The final dimensions of the construction are 6 x 6 x 1.5 cm (LxWxH). This sensor picks up vibration and with the use of proper signal processing techniques that were developed we can estimate the heart rate and the respiration rate of the patient. The load cell, the humidity sensor and the pressure-sensitive mattress are all located under the bed's mattress (Figure 5(1)) where we can achieve correct measurements without compromising comfort.

Finally, as mentioned earlier the real-time data collected by the sensors are processed and displayed on a touchscreen interface located near the patient's bed (Figure 5(3)). The system's specialized algorithms utilize this data to identify potential health issues and pressure ulcer risks, providing visual information as shown in Figure 4 and generating warnings in case of health deterioration (such us fever, tachypnea, tachycardia, ulcer, etc.).

To ensure efficient information flow for immediate clinical intervention, the system is configured to send data and early warning notifications to a cloud server and portable devices. This comprehensive monitoring system improves the quality of hospital care by offering an integrated platform for real-time monitoring and assessment of a patient's condition.

In summary, the system embodies an integrated, non-invasive solution for patient monitoring, leveraging a pressure-sensitive mattress a thermal camera, a humidity sensor, and a load cell. All sensor data is collated and visualized by a dedicated software running on a Raspberry Pi 4, with an Arduino UNO shield managing analog signals. Each sensor fulfills a specific function in capturing essential health parameters. The system's design also prioritizes user comfort and usability. As a versatile and adaptable solution, this patent represents a key milestone in non-invasive patient monitoring, promising potential advancements for more effective patient care in healthcare environments.

## Claims

1. A patient monitoring system comprising: a network of sensors, most of them integrated within a mattress cover, configured to measure surface pressures resulting from a patient's body position, infer vital parameters such as heart rate, respiratory rate, patient's position, body pressure at different body sites, humidity levels under the patient's body, as well as the body temperature of the patient.

2. The patient monitoring system of claim 1, wherein the system is further configured to display both the measured and inferred parameters in real-time on a bed-adaptive touchscreen.

3. The patient monitoring system of claim 1, wherein the system is further configured to process the measured and inferred parameters through medical rules and intelligent algorithms to detect possible pathological conditions and areas of prolonged pressure.

4. The patient monitoring system of claim 3, wherein the medical rules and intelligent algorithms enable early detection of pressure ulcers.

5. The patient monitoring system of claim 1, further comprising a user-friendly interface to provide visual information near the patient's bed.

6. The patient monitoring system of claim 1, wherein the system is further configured to send the measured and inferred parameters, data, and early warning notifications to a cloud server and portable devices, enabling clinicians to promptly identify and intervene in potential risk situations for patients.

7. The patient monitoring system of claim 1, wherein the system's functionality transforms a standard hospital bed into an intelligent patient care station, thereby improving the quality of hospitalization and eliminating the possibility of developing pressure ulcers.
